# EUROPEAN PATENT SPECIFICATION

(11) **EP 4 248 883 B1**
(45) Date of publication and mention of the grant of the patent: **26.11.2025**
(21) Application number: 20962415.4
(22) Date of filing: 18.11.2020
(51) Int. Cl.: A61B 17/062, A61B 17/29, A61B 17/00

(54) **SURGICAL INSTRUMENT**
CHIRURGISCHES INSTRUMENT
INSTRUMENT CHIRURGICAL

(43) Date of publication of application: 27.09.2023
(73) Proprietor: Nomura Unison Co., Ltd., Chino-shi, Nagano 391-0001 (JP)
(72) Inventor: KANZAWA, Hiroki, Chino-shi, Nagano 391-0001 (JP); WADA, Kenji, Chino-shi, Nagano 391-0001 (JP); TEZUKA, Chikao, Shiojiri-shi, Nagano 399-0702 (JP); SATO, Atsushi, Ina-shi, Nagano 396-8555 (JP)
(74) Representative: Liesegang, Eva
(86) International application number: PCT/JP2020/043030
(87) International publication number: WO 2022/107251

(56) References cited:
- EP-A1- 2 532 315
- WO-A1-2019/096835
- JP-A- 2010 503 457
- JP-A- 2012 045 031
- JP-A- 2013 188 323
- JP-A- 2019 025 105
- JP-A- 2019 025 105
- JP-A- H07 275 253
- US-A1- 2015 230 814
- US-A1- 2020 222 218
- US-B1- 6 217 587
- US-B2- 9 700 289

## Description

### Technical Field

The present invention relates to a surgical instrument used in a surgical operation.

### Background Art

In conducting a surgical operation, there has been known a holding device that is used for holding an extremely small object. As one example of the holding device, there has been known a needle holder for holding an anastomosis needle (see patent literature 1, for example).

Although not illustrated in the drawings, the needle holder that forms a surgical instrument described in patent literature 1 (hereinafter referred to as a conventional surgical instrument) includes: a treatment instrument that includes a pinching portion formed of a pair of pinching members whose distal ends and proximal ends are opened/closed, with a shaft used as a center; a barrel that includes a rotational cylinder portion having an opening/closing mechanism for opening/closing the distal ends of the pair of holding members and a rotational mechanism for rotationally supporting the treatment instrument; and a grip that is mounted on the rotational cylinder portion of the barrel with inclination at a prescribed angle, and includes a trigger for operating the opening/closing mechanism. Further, the conventional surgical instrument has a so-called normally closed structure where the distal ends of the holding members are closed when the trigger is not pulled, and the distal ends are opened when the trigger is pulled.

According to the conventional surgical instrument, the holding member is made to approach to an object in parallel with pulling the trigger using a middle finger and, then, the pulling of the middle finger is gradually returned so as to return the trigger to an initial position thus holding the object. In a state where the object is held in this manner, a rotational cylinder portion is sandwiched between an index finger and a thumb, and the held object can be rotated in a desired direction with these two fingers. Accordingly, for example, a user can perform a fine delicate operation such as moving of a needle that follows bending of an anastomosis needle.

### Citation List

### Patent Literature

PTL 1: JP-A-2019-25105

US 2015/230814 A1 discloses a medical instrument with a handle arranged at the proximal end, with a shank arranged on the handle, and with a tool arranged at the distal end of the shank. The medical instrument can provide several functionalities, for example the rotation about the longitudinal axis of the medical instrument, the pivoting of the tool relative to the longitudinal axis of the medical instrument and/or the actuation of the tool, for example the opening or closing of scissors. Some of these functionalities are activated with the aid of a single common actuation element. By moving the actuation element between several switch positions, different functionalities are selected, and these are activated, e.g. by rotation of the actuation element.

US 2020/222218 A1 discloses a multipurpose handle incorporated into a medical device delivery system.

US 6,217,587 B1 discloses a treatment tool for an endoscope.

WO 2019/096835 A1 discloses a medical instrument that is connected to at least one first magnetic element and having an actuating apparatus that contains at least one second magnetic element or is connected thereto.

### Summary of Invention

### Technical Problem

In operations conducted in plastic surgery, and orthopaedic surgery, it is often the case where the above-mentioned surgical procedure is conducted at a part of a human body relatively shallow from a surface of the human body. Accordingly, for example, even in an operation that requires a fine manipulation such as lymphaticovenous anastomosis, a conventional surgical instrument can be effectively used.

On the other hand, in an operation in a field of neurosurgery or the like, it is often the case where an operation is conducted at a part of a human body relatively deep from a surface of the human body as a surgical field.

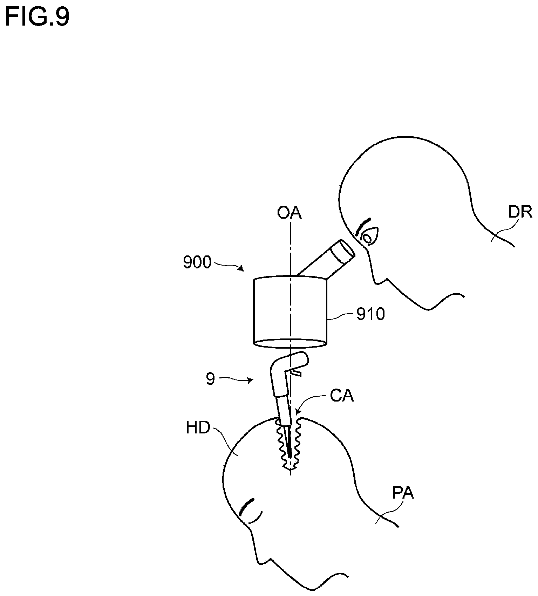

FIG.9 is a schematic view illustrating a mode where it is assumed that an operation in the field of neurosurgery is conducted using a conventional surgical instrument 9 (needle holder). In the operation in the field of neurosurgery or the like, for example, as illustrated in FIG. 9, a portion of a head HD of a human body (patient PA) is opened, the surgical instrument 9 is inserted into a narrow and deep hole CA formed in the head HD from above, an optical axis OA of an objective lens 910 of a microscope 900 is directed in the substantially same direction as the insertion direction of the surgical instrument 9, and a surgical procedure is conducted while visually observing a surgical field in the vicinity of the surgical instrument 9 under a microscope.

**In** such an operation in a field of neurosurgery, the surgical instrument is inserted from above along the optical axis OA just under the microscope 900. Accordingly, a doctor DR manipulates the surgical instrument 9 in a state where his/her wrist is free or in a state where his/her hand is likely to move or shake. Further, a gripping portion of the surgical instrument 9 used in an operation in a field of neurosurgery or the like (the barrel portion of the needle holder described in patent literature 1 corresponding to such a gripping portion) is relatively long. Accordingly, the movement or the shaking of the hand is liable to be amplified at the distal end of the treatment instrument.

Further, in the conventional surgical instrument 9, as described previously, opening/closing of the distal end of the treatment instrument is performed by manipulating the trigger using a middle finger. However, the middle finger is moved by using the same muscles (tendons) used for moving an index finger or a ring finger. Accordingly, it is considered that the doctor DR is not good at moving only the middle finger independently in the same manner as the ring finger. When the doctor DR intends to move only the middle finger independently and finely, the doctor DR moves his/her muscles (tendons) distributed in a wide range in his/her hand by delicately bringing the surgical instrument 9 into contact with the muscles (tendons). As a result, such a manipulation of the surgical instrument 9 becomes a manipulation that imposes so much strains on his/her hand and hence, his/her palm or wrist that forms a base for fingers that conduct an accurate operation is liable to move or shake. Due to the reason described above, when the treatment instrument is opened or closed in the conventional surgical instrument 9, the distal end is liable to move or shake and hence, the doctor DR cannot conduct a fine surgical procedure.

For reference, with respect to an operation in a field of plastic surgery or orthopaedic surgery, as described above, it is often the case where the surgical procedure is conducted in a part of a human body relatively shallow from a surface of the human body. Accordingly, even when the conventional surgical instrument 9 is used, an angle of the needle holder in the longitudinal direction can be largely set with respect to the visually observing direction and hence, a field of view in the vicinity of a surgical field can be relatively easily ensured. Further, in view of an angle at which the doctor DR holds the needle holder, the doctor can fix his/her wrist in the vicinity of the surgical field and hence, it is possible to obtain an environment that the manipulation of his/her hand minimally moves or shakes.

The present invention has been made in view of the above-mentioned circumstances, and it is an object of the present invention to provide a surgical instrument that can conduct a more accurate and finer surgical procedure compared to the prior art.

### Solution to Problem

The invention is defined by claim 1. Further embodiments of the invention are defined by the dependent claims.

According to one aspect of the present disclosure, there is provided a surgical instrument that includes: a treatment unit formed of a pair of treatment members having a distal end that is openable and closable; a rod-shaped body having a rod shape, the rod-shaped body having a distal end side that is connected to the treatment unit, and the rod-shaped body extending along a longitudinal direction line; a fixed cylindrical portion having an approximately cylindrical shape, the fixed cylindrical portion having a hole formed on the distal end side of the fixed cylindrical portion, the hole allowing a rear end side of the rod-shaped body to rotatably engage with the fixed cylindrical portion about the longitudinal direction line by way of an inner wall of the hole; and a gripping portion integrally provided with the fixed cylindrical portion as a portion that a user grips.

Such a surgical instrument further includes a slider handle that is mounted on an outer peripheral surface of the rod-shaped body. In the surgical instrument, the slider handle is configured to slide frontward and backward relative to the rod-shaped body (a push rod being excluded here) in the longitudinal direction line, and to rotate about the longitudinal direction line. Further, in the surgical instrument, the pair of treatment members of the treatment unit is opened or closed by moving the slider handle forward or backward along the longitudinal direction line, and the treatment unit is rotatable about the longitudinal direction line by rotating the slider handle about the longitudinal direction line.

### Advantageous effects of Invention

According to the present invention, it is possible to provide the surgical instrument that can perform a more accurate and finer surgical procedure than the prior art.

### Brief Description of Drawings

FIG. 1 is a perspective view of a surgical instrument 1 according to an embodiment 1.
FIG. 2 is a cross-sectional view of the surgical instrument 1 according to the embodiment 1.
FIG. 3 is an explanatory view of a main part for describing the structure relating to a first connecting portion of the embodiment 1.
FIG. 4 is a view illustrating a structure relating to a second connecting portion of the embodiment 1.
FIG. 5 is a perspective view of a main part illustrating the structure relating to the second connecting portion of the embodiment 1.
FIG. 6 is a view illustrating a structure relating to an open/close converting part of the embodiment 1.
FIG. 7 is a perspective view illustrating a use example of the surgical instrument 1 according to the embodiment 1.
FIG. 8 is a perspective view of a surgical instrument 2 according to an embodiment 2.
FIG. 9 is a schematic view illustrating a mode where it is assumed that a conventional surgical instrument 9 is used in an operation in a field of neurosurgery.

### Description of Embodiments

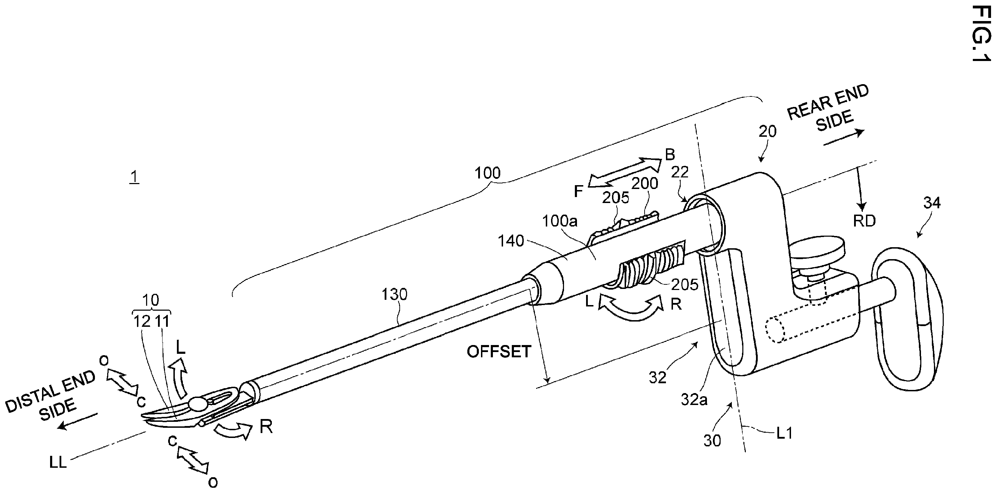

Hereinafter, embodiments of a surgical instrument 1 according to the present invention are described with reference to drawings. The respective drawings are schematic views that each indicate one example, and do not necessarily strictly reflect actual sizes, ratios and the like. Although FIG. 1 to FIG. 7 are used for illustrating the embodiment 1, these drawings are also drawings illustrating a specific example of the present invention. Accordingly, in describing the specific example of the embodiment 1, there may be a case where the embodiment 1 is simply referred to as "embodiment".

### [Embodiment 1]

### 1. Configuration of surgical instrument 1 according to embodiment 1

### (1) Overall structure of surgical instrument 1

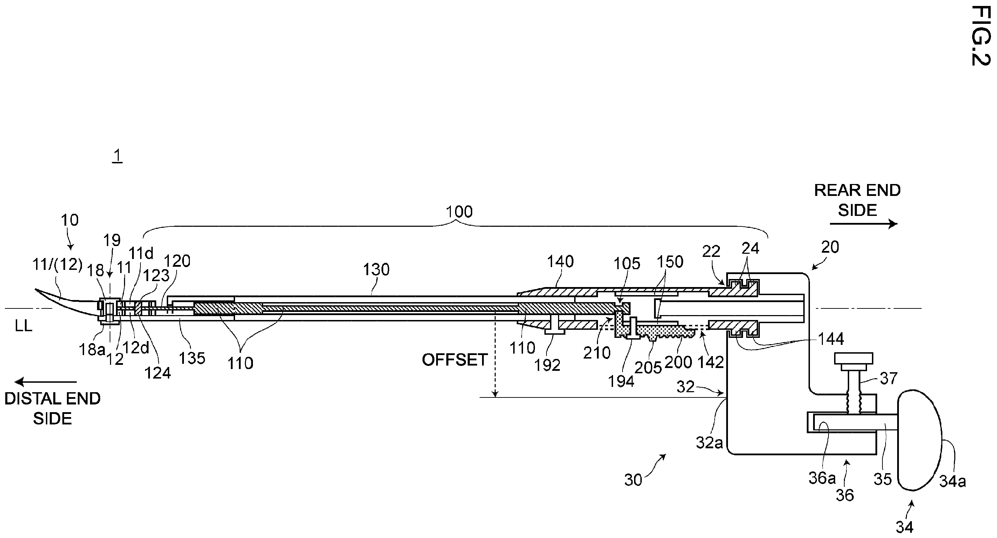

FIG. 1 is a perspective view of a surgical instrument 1 according to the embodiment 1. FIG. 2 is a cross-sectional view of the surgical instrument 1 according to the embodiment 1. A plane that includes a direction that treatment members 11, 12 are bent and a plane that includes a longitudinal direction line LL, and a plane that includes a gripping portion 30 and the longitudinal direction line LL are not necessarily the same plane. However, in FIG. 2, these planes are illustrated in the same drawing assuming that these planes are the same plane.

As illustrated in FIG. 1 and FIG. 2, the surgical instrument 1 according to the embodiment 1 includes, on the whole, a treatment unit 10, a rod-shaped body 100, a fixed cylindrical portion 20, a gripping portion 30, and a slider handle 200. In this specification, The term "longitudinal direction line LL" indicates a line along a longitudinal direction of the rod-shaped body 100, and is an imaginary line that forms a center axis when the rod-shaped body 100 rotates. The term "below" approximately indicates a direction that the gripping portion 30 protrudes from the longitudinal direction line LL of the rod-shaped body 100, and the term "above" indicates a direction opposite to the term "below". The terms "above" and "below" are terms used for convenience's sake. Accordingly, in the use of the surgical instrument 1, the term "above" and the term "below" do not necessarily agree with the up-down direction along a vertical line.

### (2) Treatment unit 10

The treatment unit 10 includes a pair of treatment members 11, 12 that has openable and closable distal ends.

The treatment unit 10 may have any structure provided that the treatment unit 10 is divided into at least two members, and both distal ends are openable and closable about an open/close shaft 19 (see FIG. 2). In such a configuration, the description is made hereinafter by taking a needle holder that holds an anastomosis needle as an example.

The treatment unit 10 is not limited to the needle holder. Tweezers or forceps for gripping or grasping an object and scissors for cutting the object are also preferably used as the treatment unit 10. That is, the treatment unit 10 may be the needle holder, the tweezers or the forceps. Further, the treatment unit 10 may be scissors.

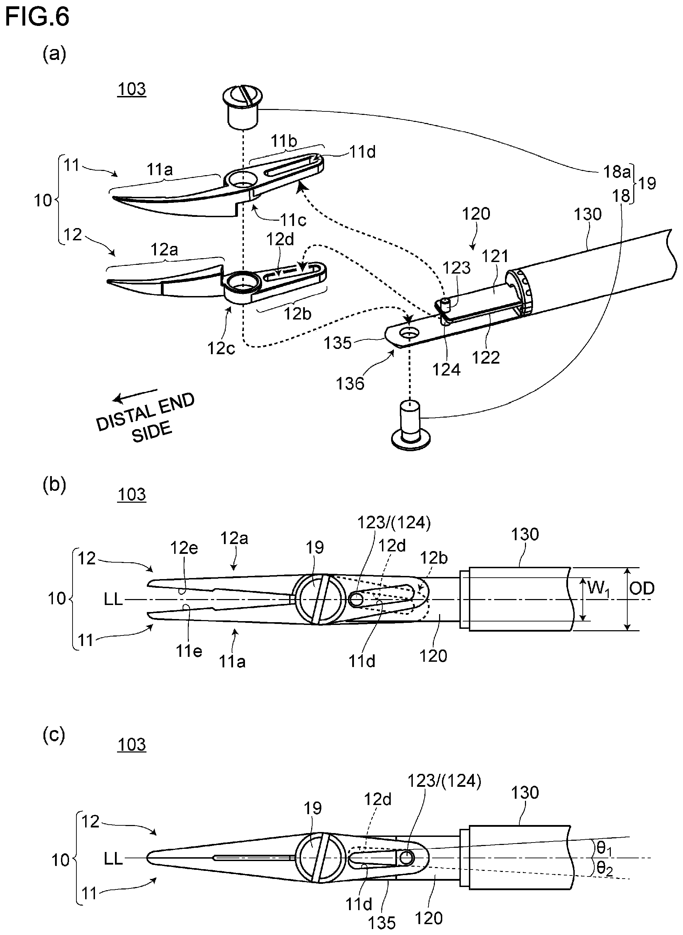

In the embodiment 1, the distal end of the treatment unit 10 is located at the position away from the longitudinal direction line LL in the radial direction. In this embodiment, as the distal end sides of the treatment members 11, 12 extend toward distal ends of the treatment members 11, 12 from the open/close shaft 19, the distal end sides of the treatment members 11, 12 are bent so as to be away from the longitudinal direction line LL. However, the distal end of the treatment unit 10 is not limited to such a configuration. For example, the distal end of the treatment unit 10 may be formed into an approximately L shape from middle portions of the treatment portions 11a, 12a (see FIG. 6(a) described later).

### (3) Rod-shaped body 100

The rod-shaped body 100 connects the treatment unit 10 and the fixed cylindrical portion 20 (described later) to each other, and is referred to an entity of a rod-shaped member extending along the longitudinal direction line LL. The rod-shaped body 100 includes: a hollow pipe 130 described later; a push rod 110 that is made to pass through the inside of the hollow pipe 130; a barrel 140 that is connected to an outer peripheral surface of the hollow pipe 130 and the like. The treatment unit 10 is connected to a distal end side of the rod-shaped body 100, and the fixed cylindrical portion 20, the gripping portion 30 and the like described later are disposed on a rear end side of the rod-shaped body 100. Although described in detail later, the treatment unit 10 engages with the distal end of the push bar 110 by way of a flat plate 120 and an upper boss 123 and a lower boss 124 that protrude from the flat plate 120. Further, in the inside of the barrel 140, a hollow member 150 is disposed in such a manner that the hollow member 150 is advanceable and retractable along the longitudinal direction line LL and is rotatable about the longitudinal direction line LL. The hollow member 150 is rotatable such that a center axis of the hollow member 150 agrees with the longitudinal direction line LL, and is movable in the longitudinal direction along the longitudinal direction line LL.

In this specification, the term "distal end side" is referred to as a side where the treatment unit 10 is disposed with respect to the rod-shaped body 100, and the term "rear end side" is referred to as a side where the fixed cylindrical portion 20 is disposed with respect to the rod-shaped body 100.

### (4) Fixed cylindrical portion 20

The fixed cylindrical portion 20 is an approximately cylindrical portion that has a hole 22 formed on a distal end side of the fixed cylindrical portion 20. Guide grooves 24 (see FIG. 2) are formed on an inner wall of such a hole 22 in a circumferential direction. On the other hand, flange portions 144 having a flange shape that protrude in a radial direction RD from the longitudinal direction line LL are formed on a rear end side of the barrel 140 that forms the rod-shaped body 100. The flange portions 144 engage with the guide grooves 24 formed on the fixed cylindrical portion 20. In other words, the fixed cylindrical portion 20 is formed such that a rear end side of the rod-shaped body 100 (more specifically, a rear end side of the barrel 140) rotatably engages with the fixed cylindrical portion 20 about the longitudinal direction line LL by way of an inner wall of the hole 22.

In this embodiment, the shape of an opening portion of the fixed cylindrical portion 20 is formed into an approximately circular shape. However, so long as the fixed cylindrical portion 20 and the rod-shaped body 100 are rotatable, eventually, the shape of the opening portion of the fixed cylindrical portion 20 is not particularly limited to an approximately circular shape.

### (5) Gripping portion 30

The gripping portion 30 is a portion that is integrally provided with the fixed cylindrical portion 20 as a portion that a user of the surgical instrument 1 grips. In the embodiment 1, the gripping portion 30 is formed in a protruding manner toward the radial direction RD from the longitudinal direction line LL. The fixed cylindrical portion 20 and the gripping portion 30 may be integrally formed (continuously formed) with each other, or the fixed cylindrical portion 20 and the gripping portion 30 are separately formed from each other and may be connected to each other.

To describe the gripping portion 30 of the embodiment 1 in detail, the gripping portion 30 has at least a finger engaging portion 32 and a palm contact portion 34.

The finger engaging portion 32 includes a finger engaging surface 32a that is a surface formed for allowing a finger of a user to engage with the finger engaging portion 32. The direction of a tangent line L1 of the finger engaging surface 32a is set such that the direction intersects with the longitudinal direction line LL. In other words, the tangent line of the finger engaging surface 32a is not parallel to the longitudinal direction line LL. In the embodiment, the tangent line of the finger engaging surface 32a is orthogonal to the longitudinal direction line LL.

The finger engaging portion 32 is disposed at the position offset from the longitudinal direction line LL in the radial direction of the rod-shaped body 100 by a predetermined size (see FIG. 2). An offset amount of the finger engaging portion 32 is set corresponding to a size of a palm of a user or the like. It is preferable that the offset amount be set within a range of approximately 5mm to 50mm.

The finger engaging portion 32 is a portion with which at least a middle finger of a user engages. However, a finger that engages with the finger engaging portion 32 is not limited to the middle finger, and the finger engaging portion 32 may be formed as a portion with which a ring finger also engages in addition to the middle finger. With such a configuration, in the use of the surgical instrument 1, the gripping portion 30 can be gripped in such a manner that the gripping portion 30 is sandwiched between two fingers consisting of the middle finger and the ring finger and the palm. Accordingly, the gripping portion 30 and the fixed cylindrical portion 20 which form a base in manipulating the surgical instrument 1 can be held more stably and, further, the surgery can be performed with more accurate procedure (method of using the surgical instrument 1 being described later).

The palm contact portion 34 is a portion that is brough into contact with a palm of a user when the user grips the surgical instrument 1. The palm contact portion 34 is disposed on a rear end side as viewed from the finger engaging portion 32. Although the palm contact portion 34 can take any suitable shape, in this embodiment, the palm contact portion 34 has a hemispherical shape (a shape similar to a mouse of a personal computer) that is obtained by dividing a rugby ball in halves. That is, the palm contact portion 34 is arranged such that an elongated curved surface portion of the palm contact portion 34 that is substantially parallel to a major axis of the hemispherical shape is brought into contact with the palm as a palm contact surface 34a.

The palm contact portion 34 may be fixedly formed on the finger engaging portion 32. However, it is preferable that a distance between the palm contact portion 34 and the slider handle 200 (in the direction along the longitudinal direction line LL) can be changed.

In the embodiment, on one hand, a stay 35 is continuously formed on the palm contact portion 34 on a side opposite to the palm contact surface 34a. On the other hand, a gripping portion intermediate portion 36 is formed on a rear end side of the gripping portion 30 in the longitudinal direction line LL in a protruding manner from the finger engaging portion 32, a stay hole 36a that opens on a rear end side of the gripping portion intermediate portion 36 is formed, and the stay 35 is inserted into the stay hole 36a in an advanceable and retractable manner. With such configuration, the distance between the palm contact portion 34 and the finger engaging surface 32a can be changed and hence, the distance between the palm contact portion 34 and the slider handle 200 can be changed. In this embodiment, by fastening a stopper screw 37 at a stage that the distance is set, the position of the palm contact portion 34 can be fixed.

In this manner, the distance between the palm contact portion 34 and the slider handle 200 can be changed. Accordingly, the distance can be adjusted such that a user can easily grip the surgical instrument 1 corresponding to a shape, a size and the like of a palm of the user and hence, the user can perform the more accurate and finer surgical procedure.

The shape of the palm contact portion 34 may be a shape that resembles a hemispherical shape obtained by dividing a chicken egg into halves, or may be a semispherical shape obtained by dividing a true sphere into halves, or may be an approximately plate shape such as a shape that resembles first to third bases used in baseball, for example. In a case where the palm contact portion 34 is a hemispherical shape obtained by dividing an egg of a chicken into halves (not illustrated in the drawings), the stay 35 may be formed on a back side of an offset protruding portion that protrudes on a rear end side in an offset manner. In this case, by rotating the stay 35 in the stay hole 36a at a suitable angle and by fixing the stay 35, a part of a user that falls within a region of his/her palm and is brought into contact with the palm contact surface 34a can be changed. With such a configuration, the user can grip the surgical instrument 1 more accurately in conformity with the shape, the size or the like of the palm of the user.

### (6) Slider handle 200

The slider handle 200 is a portion that is manipulated by the user for performing the rotation of the distal end of the treatment unit 10 and opening or closing of such a distal end.

The slider handle 200 is mounted on an outer peripheral surface 100a of the rod-shaped body 100. The slider handle 200 may be mounted such that the slider handle 200 is brough into contact with an outer peripheral surface 100a of the rod-shaped body 100. Alternatively, the slider handle 200 may be mounted outside the outer peripheral surface 100a in a radial direction in a state where the slider handle 200 is slightly lifted from the outer peripheral surface 100a so as to prevent the slider handle 200 from being brought into contact with the outer peripheral surface 100a. It must be noted that such modes are also included in "mounted". The slider handle 200 may be formed so as to be wrapped around the outer peripheral surface 100a of the rod-shaped body 100 by an approximately half of the entire circumference (the case described in the embodiment), or may be formed so as to be simply wrapped around only a portion of the outer peripheral surface 100a of the rod-shaped body 100 as in the case of a commercially available slide switch, or may be formed so as to be wrapped around an almost entire circumference or a completely entire circumference of the outer peripheral surface 100a. In the case where the slider handle 200 is formed so as to be wrapped around an almost entire circumference or a completely entire circumference of the outer peripheral surface 100a, even when the slider handle 200 is rotated with a large angle, it is possible to provide the surgical instrument where fingers are minimally removed from the slider handle 200.

Because of the structure formed of a first connecting portion 101, a second connecting portion 102 and the like described later, the slider handle 200 slides in the longitudinal direction along the longitudinal direction line LL with respect to the rod-shaped body 100 (excluding the push rod 110 (described later)), and rotates about a longitudinal direction line LL.

The center axis of the rotation of the slider handle 200 and the center axis of the rod-shaped body 100 (at least a portion of the rod-shaped body 100 in the vicinity of a position where the slider handle 200 is mounted) have the coaxial relationship. Further, the direction of the longitudinal movement of the slider handle 200 is the same direction as the longitudinal direction line LL.

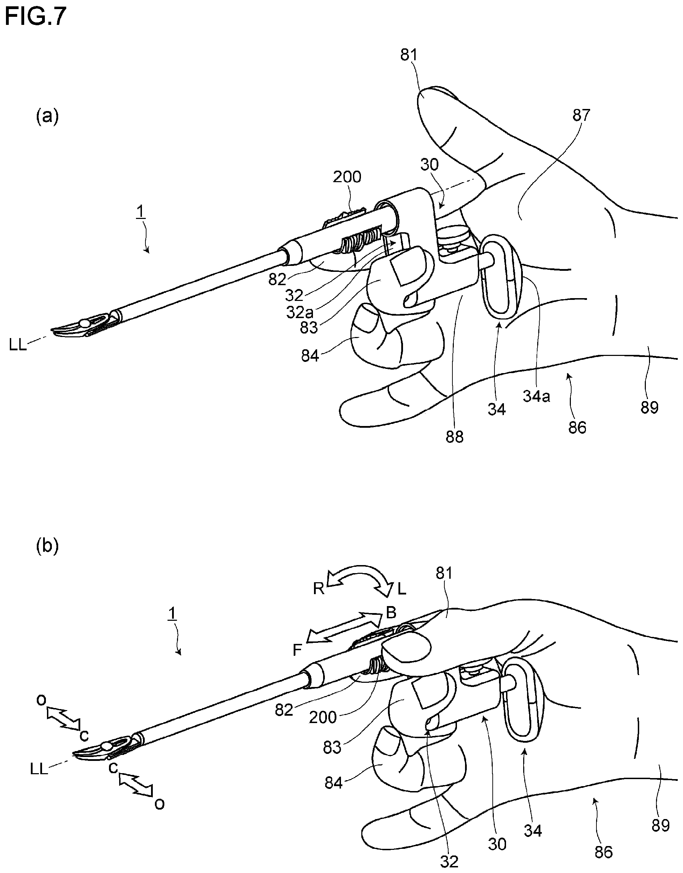

The slider handle 200 is manipulated by a thumb 81 and an index finger 82 of a user (also see FIG. 7(b) described later). More specifically, the slider handle 200 is a portion where the slider handle 200 is brought into contact with the user in such a manner that the slider handle 200 is sandwiched between a ball of the thumb 81 and a ball of the index finger 82, and the slider handle 200 is manipulated by these both fingers. The above-mentioned use mode also includes a use mode where either one of the thumb 81 or the index finger 82 is used for the manipulation, and the other of the thumb 81 or the index finger 82 is merely brought into contact with the handle. Further, the above-mentioned use mode also includes a use mode where the user performs the manipulation by bringing only either one of the thumb 81 or the index finger 82 depending on the user.

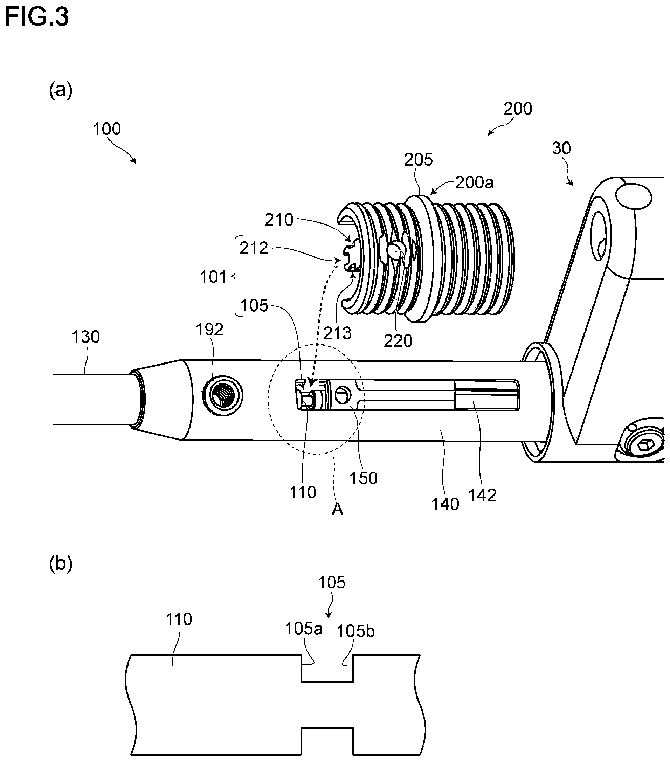

A corrugated concavo-convex portion that functions as a slip stopper is formed on a surface of the slider handle 200. Further, it is preferable that a position marker 205 be formed on a surface 200a of the slider handle 200. Provided that the position marker 205 becomes a mark indicating the position and the posture of the slider handle 200 to a user when the user touches the slider handle 200, the position marker 205 may take any configuration. In the embodiment, the position marker 205 is formed of a reference protrusion that protrudes higher than the above-mentioned corrugated concavo-convex portion by one stage (see also FIG. 3 described later). However, the position marker 205 is not limited to such a configuration, and may be formed of a recess as an opposite case, or may be formed of a hole or the like.

By forming the position marker 205 on the slider handle 200, a user can manipulate the surgical instrument 1 while recognizing the relative positional relationship or the like of the slider handle by making use of the position marker 205. Accordingly, the user can perform the surgical procedure more accurately.

The surgical instrument 1 according to the embodiment 1 includes: the treatment unit 10, the rod-shaped body 100, a fixed cylindrical portion 20, and the gripping portion 30. Further, the surgical instrument 1 includes the slider handle 200 that is mounted on the outer peripheral surface 100a of the rod-shaped body 100, and is configured to slide along the longitudinal direction line LL with respect to the rod-shaped body 100 and to rotate about the longitudinal direction line LL. Such a surgical instrument 1 is configured such that, the treatment members 11, 12 of the treatment unit 10 that form a pair are opened or closed by advancing or retracting the slider handle 200 along the longitudinal direction line LL, and the treatment unit 10 is rotated about the longitudinal direction line LL by rotating the slider handle 200 about the longitudinal direction line LL. In the embodiment, when the slider handle 200 is advanced in the direction indicated by a bold arrow F, the pair of treatment members 11, 12 are opened in directions indicated by a bold arrow O in an interlocking manner with the advancing of the slider handle 200. On the other hand, when the slider handle 200 is retracted in the direction indicated by a bold arrow B, the pair of treatment members 11, 12 are closed in directions indicated by a bold arrow C in an interlocking manner with the retracting of the slider handle 200. Further, when the slider handle 200 is rotated in the direction indicated by R, the entire treatment unit 10 is rotated in the direction indicated by the bold arrow R in an interlocking manner with such rotation of the slider handle 200. On the other hand, when the slider handle 200 is rotated in the direction indicated by L, the entire treatment unit 10 is rotated in the direction indicated by the bold arrow L in an interlocking manner with such rotation of the slider handle 200 (see FIG. 1).

The description of surgical instrument 1 of the embodiment is continued hereinafter by focusing on the specific configuration for realizing such an operation.

### (7) First connecting portion 101

As described above, the rod-shaped body 100 includes the push rod 110 having the distal end with which the treatment unit 10 engages. The "push rod 110" has not only a function of pushing the flat plate 120 (described later) but also a function of pulling the flat plate 120 toward a rear side.

The surgical instrument 1 includes the first connecting portion 101. The first connecting portion 101 connects the slider handle 200 and the push rod 110 to each other. The first connecting portion 101 is provided for converting the longitudinal movement of the slider handle 200 into the longitudinal movement of the push rod 110.

FIG. 3 is an explanatory view of a main part for describing the structure relating to the first connecting portion 101 of the embodiment 1. FIG. 3(a) is an exploded view of the main part of a portion of the surgical instrument 1 as viewed from an oblique lower side. In the drawings, a state is illustrated where the slider handle 200 is removed from the barrel 140. A screw 194 for fixing the slider handle 200 is not illustrated in the drawing. FIG. 3(b) is a view illustrating a cross section of the push rod 110 that corresponds to a region surrounded by a broken line A in FIG. 3(a).

As illustrated in FIG. 3, the slider handle 200 is formed into an approximately semicircular cylindrical shape obtained by dividing a circular cylinder in halves along an axial direction. A pawl 210 is formed on an inner side of the slider handle 200. The pawl 210 protrudes toward a direction of a rotation axis (the longitudinal direction line LL in a mounted state) when the slider handle 200 is rotated from the position of an edge of the slider handle 200 on a distal end side. A distal end portion 212 of the pawl 210 is formed into a circular arcuate shape following a shape of a periphery of a guide groove 105 (described later). When the slider handle 200 is rotated, the distal end portion 212 slides while being guided along a periphery of the guide groove 105 (described later).

The position at which the pawl 210 is mounted in the thrust direction (the direction along the longitudinal direction line LL) is not limited to the edge on the distal end side, and may be disposed at an intermediate position between the distal end side and a rear end side of the slider handle 200.

On the other hand, in the rod-shaped body 100, the push rod 110 is housed in inner spaces of the hollow pipe 130, the barrel 140 and the like. In FIG. 3(a), the push rod 110 can be slightly checked through a slit 142 formed in the barrel 140. As illustrated in FIG. 3(b) that is a cross-sectional view, the guide groove 105 is formed on the push rod 110 on a rear end side (see also FIG. 2). The guide groove 105 formed on the push rod 110 is a groove having a diameter smaller than other portions of the push rod 110, and is formed in a circumferential direction. The distal end portion 212 of the pawl 210 of the slider handle falls within the guide groove 105 and engages with the guide groove 105.

The slit 140 is formed in the barrel 140. When the slider handle 200 is combined with the push rod 110 through the slit 142, the distal end portion of 212 of the pawl 210 of the slider handle engages with and is connected to the guide groove 105 formed in the push rod 110. When the slider handle 200 is moved in the longitudinal direction along the thrust direction in such a state, a portion of the pawl 210 of the slider handle 200 in the vicinity of the distal end portion 212 pushes the inner walls 105a, 105b of the guide groove 105 (see FG. 3(b)). Accordingly, the entire push rod 110 also moves in the longitudinal direction along the thrust direction.

From the above description, it is understood that the first connecting portion 101 is formed of: the distal end portion 212 of the pawl 210 of the slider handle: and the guide groove 105 formed on the push rod 110.

### (8) Second connecting portion 102

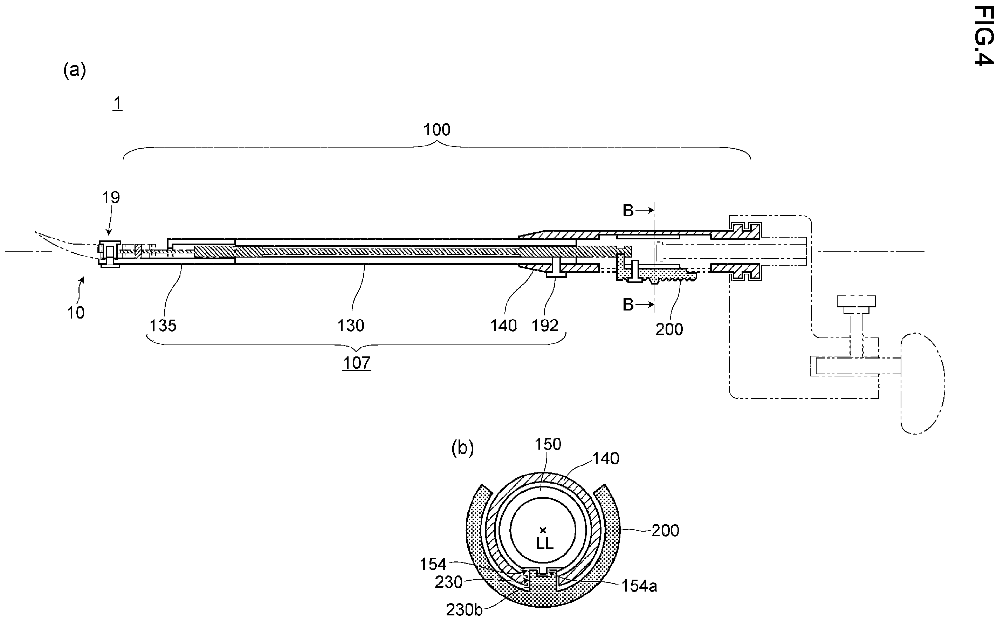

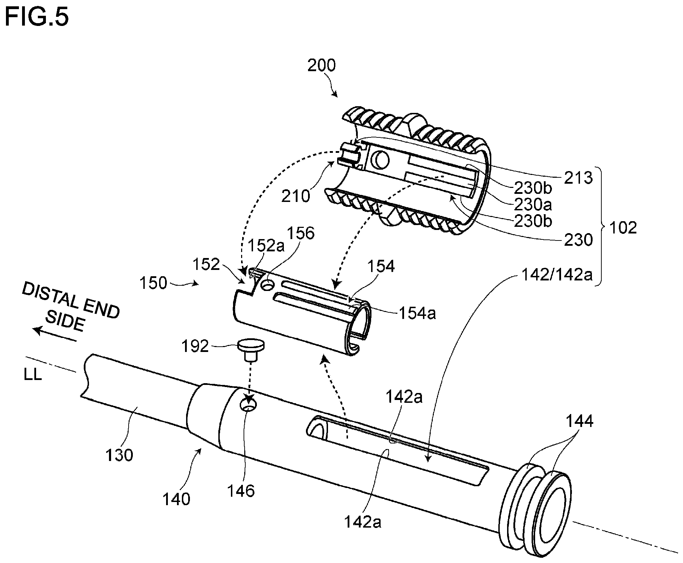

FIG. 4 is a view for describing the structure relating to the second connecting portion 102 according to the embodiment 1. FIG. 4(a) is a cross-sectional view for describing a fixing portion 107 of the rod-shaped body 100 for fixing the open/close shaft 19. Fig. 4(b) is a cross-sectional view taken along B-B in FIG. 4(a)). FIG. 5 is a perspective view of a main part illustrating the structure relating to the second connecting portion 102 of the embodiment 1. In FIG. 5, the illustration of a portion of the rod-shaped body 100 on a more distal end side than an intermediate portion of the rod-shaped body 10, the fixed cylindrical portion 20, the gripping portion 30 and the like is omitted. Further, a state is illustrated where the hollow member 150 and the slider handle 200 are removed from the barrel 140.

As illustrated in FIG. 4 and FIG. 5, the surgical instrument 1 includes the second connecting portion 102 (see FIG. 5 with respect to symbol 102).

The second connecting portion 102 connects the slider handle 200 and the "fixing portion 107 of the rod-shaped body 100 to which the open/close shaft 19 of the treatment unit 10 is fixed" to each other. The second connecting portion 102 is provided for converting the rotation of the slider handle 200 into the rotation of the treatment unit 10.

In the above-mentioned configuration, the fixing portion 107 of the rod-shaped body 100 to which the open/close shaft 19 of the treatment unit 10 is fixed may be formed of any member provided that the open/close shaft 19 is fixed to the fixing portion 107. In the embodiment 1, as illustrated in FIG. 4(a), "the fixing portion 107 of the rod-shaped body 100 that fixes the open/close shaft 19" is formed of: a treatment unit base 135 that supports the open/close shaft 19 from a distal end side; the hollow pipe 130 having a distal end to which the treatment unit base 135 is joined; and the barrel 140 that allows the hollow pipe 130 to pass through the barrel 140 and is joined to the hollow pipe 130 by the screw 192.

As illustrated in FIG. 5, on an inner side of the slider handle 200, an engaging protruding ridge portion 230 that protrudes in an elongated manner toward the direction of the center axis of rotation of the slider handle 200 is formed. An inner groove 230a is also formed in the vicinity of the center of the engaging protruding ridge portion 230. On the other hand, an engaging groove 154 is formed on an outer side of the hollow member 150. Further, an inner protrusion 154a that follows the shape of the above-mentioned inner groove 230a is formed in the vicinity of the center of the engaging groove 154. With such a configuration, in a state where the surgical instrument 1 is assembled, the engaging protruding ridge portion 230 of the slider handle 200 engages with the engaging groove 154 formed in the hollow member 150 through the slit 142 formed in the barrel 140 (also see FIG. 4(b)).

Further, the slider handle 200 is fastened to the hollow member 150 by making the screw 194 (the illustration of the screw 194 being omitted in FIG. 5) threadedly engage with a threaded hole 156 formed in the hollow member 150 through the slit 142 formed in the barrel 140.

With the configurations described above, the slider handle 200 is joined to the rod-shaped body 100 without being removed from the rod-shaped body 100.

For reference, the pawl 210 of the slider handle passes through the cutout portion 152 of the hollow member 150 and hence, the pawl 210 does not interfere with the hollow member 150.

With the configuration described above, when the slider handle 200 is rotated, a side surface 230b of the engaging protruding ridge portion 230 of the slider handle 200 is brought into contact with an inner wall 142a of the slit 142 of the barrel 140 so that the entire barrel 140 is also rotated in a form that the side surface 230b pushes the inner wall 142a (see FIG 4(b) and FIG. 5). As described above, the barrel 140 forms a portion of "the fixing portion 107 of the rod-shaped body 100 that fixes the open/close shaft 19" and hence, the barrel 140 is indirectly joined with the open/close shaft 19, and forms an integral body together with the open/close shaft 19. Accordingly, when the barrel 140 is rotated, the open/close shaft 19 is also rotated. As a result, the treatment unit 10 whose distal end opens/closes about the open/close shaft 19 is also rotated.

From the above, it is understood that the second connecting portion 102 is formed of: the engaging protruding ridge portion 230 (more specifically, the side surface 230b) of the slider handle: and the slit 142 (more specifically, the inner wall 142a of the slit).

### (9) Open/close converting portion 103

The surgical instrument 1 includes an open/close converting portion 103. The open/close converting portion 103 is provided for converting the forward and backward movement of the push rod 110 into opening and closing of the treatment unit 10.

FIG. 6 is a view illustrating a structure relating to the open/close converting portion 103 of the embodiment 1. FIG. 6(a) is an exploded view of the open/close converting portion 103, FIG. 6(b) is a plan view of the open/close converting portion 103 in a state where the treatment unit 10 is opened, and FIG. 6(c) is a plan view of the open/close converting portion 103 in a state where the treatment unit 10 is closed.

As illustrated in FIG. 6(a), the treatment unit 10 is formed of a pair of treatment members consisting of a treatment member 11 and a treatment member 12.

A shaft hole 11c is formed in the treatment member 11 and a shaft hole 12c is formed in the treatment member 12. Each treatment member 11, 12 has a treatment portion 11a, 12a at a position closer to a distal end side than the shaft hole 11c, 12c, and has a manipulating portion 11b, 12b at a position closer to a rear end side than the shaft hole 11c, 12c. The pair of these treatment members 11, 12 are joined to each other by the open/close shaft 19 that is inserted into the shaft holes 11c,12c of the respective treatment members 11, 12 in common. In the embodiment 1, the open/close shaft 19 is formed of a pin 18 and a pin cap 18a.

Further, in each manipulating portion 11b, 12b, a cam groove 11d, 12d that makes a predetermined angle θ1, θ2 (see FIG. 6(c)) with respect to the longitudinal direction line LL when the treatment unit 10 is closed is formed. In such a configuration, it is preferable that the predetermined angles θ1, θ2 fall within a range of approximately 1 to 5° respectively. For example, it is more preferable that the predetermined angles θ1, θ2 fall within a range of approximately 2 to 3°.

On the other hand, a flat plate 120 is disposed on a distal end of the push rod 110. An upper boss 123 is formed on a first surface 121 of the flat plate 120, and a lower boss 124 is formed on a second surface 122 of the flat plate 120 that is a surface opposite to the first surface 121. The upper boss 123 and the lower boss 124 are disposed at the same position as viewed in a plan view. However, the arrangement of the upper boss 123 and the lower boss 124 is not limited to such an arrangement.

The upper boss 123 engages with the cam groove 11d formed in one treatment member 11, and the lower boss 124 engages with the cam groove 12d formed in the other treatment member 12.

With such a configuration, in a state where the push rod 110 is advanced as illustrated in FIG. 6(b), the upper boss 123 and the lower boss 124 formed on the flat plate 120 are positioned at an intersecting point of the cam grooves 11d, 12d that have the predetermined angles respectively and at distal end sides of the cam grooves 11d, 12d. In this state, the distal end sides of the manipulating portions 11b, 12b are opened about the open/close shaft 19.

Next, when the push rod 110 is retracted as illustrated in FIG. 6(c), the upper boss 123 and the lower boss 124 are positioned at an intersecting point of the cam grooves 11d, 12d that have the predetermined angles respectively and at rear end sides of the cam grooves 11d, 12d. In this state, the distal end sides of the manipulating portions 11b, 12b are closed about the open/close shaft 19. To describe the above dynamically, when a user retracts the slider handle 200, the positions of the upper boss 123 and the lower boss 124 move along the cam grooves 11d, 12d and, along with such movement of the positions, the distal ends of the treatment portions 11a, 12a shift from an open state to a closed state.

From the above, it is understood that the open/close converting portion 103 is formed of: the flat plate 120; the upper boss 123 and the lower boss 124 that are formed on the flat plate 120; the cam grooves 11d, 12d that engage with these bosses; and the pair of treatment members that have the cam grooves 11d, 12d relating to the manipulating portions.

The surgical instrument 1 adopts the configuration described heretofore. Accordingly, when a user advances or retracts the slider handle 200 along the longitudinal direction line LL, the pair of treatment members 11, 12 of the treatment unit 10 are opened or closed. Further, when the user rotates the slider handle 200 about the longitudinal direction line LL, the treatment unit 10 is rotated about the longitudinal direction line LL.

In the embodiment 1, the predetermined angles θ1, θ2 and the lengths of the cam grooves 11d, 12d are suitably set. Accordingly, as viewed in a plane perpendicular to the longitudinal direction line LL, the manipulating portions 11b, 12b of the treatment unit 10 and the flat plate 120 fall within an outer diameter OD of the hollow pipe 130 that houses the push rod 110 (indicated by a width W1 in FIG. 6(b).

Further, in the embodiment 1, a hole that has its center on the longitudinal direction line LL may be formed on a rear end of the fixed cylindrical portion 20 (the hole not illustrated in the drawing), and the hole may communicate with the hole 22 formed in a distal end side of the fixed cylindrical portion 20. By adopting such a configuration, the inside of the fixed cylindrical portion 20 may be cleaned in the same manner as the surgical instrument described in patent literature 1.

### 2. Use example and advantageous effects of surgical instrument 1 according to embodiment 1

The surgical instrument 1 according to the embodiment 1 can be suitably used in neurosurgery, for example.

### (1) Use example of surgical instrument 1

FIG. 7 is a perspective view illustrating a use example of the surgical instrument 1 according to the embodiment 1. FIG. 7(a) is a view illustrating a mode of a first step where a user starts gripping the surgical instrument 1, and FIG. 7(b) is a view illustrating a mode of a second step where the user performs the forward and backward movement and the rotation of the slider handle 200. In FIG. 7, an example where the user holds the surgical instrument 1 with his/her right hand is illustrated. When the user holds the surgical instrument 1 with his/her left hand, the user uses the surgical instrument 1 in a reversed relationship.

First, as illustrated in FIG. 7(a), a user brings the palm contact portion 34 disposed on a rear end side of the surgical instrument 1 into contact with a palm 86 of his/her right hand. Then, the user engages his/her middle finger 83 with the finger engaging portion 32 (finger engaging surface 32a). For example, when the user brings a palm contact surface 34a into contact with a portion of his/her palm in the vicinity of the center of his/her palm between a ball of his/her finger 88 and a ball of his/her thumb 87, the user can grip the surgical instrument 1 stably in such a manner that the palm contact portion 34 is sandwiched between the ball of his/her finger 88 and the ball of his/her thumb 87 (first step).

Next, the user brings his/her thumb 81 and his/her index finger 82 into contact with the slider handle 200, and grips the slider handle 200 in such a manner that the slider handle 200 is sandwiched between the thumb 81 and the index finger 82. In this case, it is desirable that the user brings the thumb 81 and/or the index finger 82 into contact with the position marker 205 (not illustrated in the drawing) so as to grasp the positional relationship.

Then, the user advances or retracts the slider handle 200 along the longitudinal direction line LL using the thumb 81 and the index finger 82 so that the distal end of the treatment unit 10 is opened or closed. The user also rotates the slider handle 200 about the longitudinal direction line LL using the thumb 81 and the index finger 82 so as to rotate the entire treatment unit 10. Accordingly, by performing only the manipulations using the thumb 81 and the index finger 82, the user (doctor) can realize an intended surgical procedure with the treatment unit 10 on a distal end side.

### (2) Advantageous effects acquired by surgical instrument 1

(2-1) As described also in the column "Technical Problem", the middle finger 83 is a finger that is not suitable for an accurate manipulation. When a user intends to perform an accurate manipulation by the middle finger 83 for opening or closing the treatment unit using the conventional surgical instrument 9, the manipulation imposes strains on the user. As a result, the palm 86 or the wrist 89 that forms the base for manipulation moves or shakes. Particularly, when an elongated surgical instrument where the rod-shaped body has a large size is used, the movement or the shaking of the hand is amplified at the distal end of the treatment unit and hence, the fine surgical procedure is impaired.

The surgical instrument 1 according to the embodiment 1 is configured such that the slider handle 200 is mounted on the outer peripheral surface 100a of the rod-shaped body 100, the slider handle 200 slides forward and backward along the longitudinal direction line LL with respect to the rod-shaped body 100, and the slider handle 200 can rotate about the longitudinal diction line LL. By moving the slider handle 200 forward and backward along the longitudinal direction line LL, the pair of treatment members 11, 12 of the treatment unit 10 are opened or closed, and by rotating the slider handle 200 about the longitudinal direction LL, the treatment unit 10 rotates about the longitudinal direction line LL.

Accordingly, as illustrated in FIG. 7(b), when the user pinches the slider handle 200 by sandwiching the slider handle 200 between the thumb 81 and the index finger 82, the user can not only rotate the treatment unit 10 by rotating the thumb 81 and the index finger 82 but also open/close the treatment unit 10 by moving the thumb 81 and the index finger 82 forward or backward (advancing or retracting).

That is, opening and closing of the treatment unit 10 can be realized without using the middle finger 83 at all and hence, there is no possibility of the occurrence of the shaking of the palm 86 or the wrist 89 that may be caused due to the above-mentioned movement of the middle finger 83.

For reference, the thumb 81 and the index finger 82 are fingers that can perform an accurate operation without having a strain compared to the middle finger 83 because of the manner of building of muscles (tendons). In the surgical instrument 1 according to the embodiment 1, the user can perform opening/closing and rotation of the treatment unit 10 using only the thumb 81 and the index finger 82 by which an accurate operation can be performed without a strain. Accordingly, the user can accurately and finely perform the manipulation of the treatment unit 10 with the manipulation of the slider handle 200.

From the above, according to the surgical instrument 1 of the embodiment 1, even when the treatment unit 10 is located at the position relatively away from his/her hand, the user can accurately and finely sandwich, pinch and rotate an object. As a result, compared to the prior art, the user can perform the fine surgical procedure (operation).

(2-2) The surgical instrument 1 according to the embodiment 1 is configured such that the finger engaging portion 32 is disposed at the position that is offset in the radial direction of the rod-shaped body 100 from the longitudinal direction line LL as the gripping portion 30, and the palm contact portion 34 is disposed on a rear end side as viewed from the finger engaging portion 32. By adopting such a configuration, the user (doctor) can engage the middle finger 83 with the finger engaging portion 32 while bringing the palm 86 into contact with the palm contact portion 34 and hence, the user can grip the surgical instrument 1 stably. Since the user can stably grip the surgical instrument 1, the user can perform the manipulation with smaller shaking of the surgical instrument 1.

(2-3) For example, the user can also easily perform an extremely fine surgical procedure such as sticking a needle into a suturing object along a curve of an anastomosis needle and rotating the needle while pinching the anastomosis needle. Further, according to the surgical instrument 1, a target that the user manipulates is limited to only the slider handle 200 and the user can easily simultaneously perform opening/closing and rotation of the treatment unit 10. The user can close/open the distal end of the treatment unit 10 in parallel with, for example, twisting of the treatment unit 10.

In the conventional surgical instrument 9, the degree of opening/closing is determined corresponding to an angle of the trigger. In general, it is difficult to control the angle of the trigger. On the other hand, in the surgical instrument 1 according to the embodiment 1, the degree of opening/closing of the treatment unit 10 is determined based on the position on one axis (longitudinal direction line). Accordingly, the fine adjustment of opening/closing of the treatment unit 10 can be easily performed and hence, the user can easily and intuitively perceive feeling of pinching the object.

(2-4) In the conventional surgical instrument 9, it is necessary to house the leaf spring and the like and hence, it is necessary to surely provide the outer diameter of the rodshape body 100 (the hollow pipe 130 or the like) with a margin by an amount corresponding to the leaf spring. On the other hand, the surgical instrument 1 according to the embodiment 1 is configured such that the surgical instrument 1 includes the open/close converting portion 103 in a mode where the bosses engage with the cam grooves 11d, 12d as described above, and the manipulating portions 11b, 12b of the treatment unit 10 and the flat plate 120 fall within the inside of an outer diameter of the hollow pipe 130 that houses the push rod 110 (see FIG. 6(b)).

Accordingly, in the surgical instrument 1 according to the embodiment 1, designing of making an outer diameter of the rod-shaped body 100 extremely small can be performed easily compared to the conventional surgical instrument 9. By making the outer diameter of the rod-shaped body 100 extremely small, for example, when a user intends to watch the distal end of the treatment unit 10 using the microscope 900 in an operation in neurosurgery or the like as illustrated in FIG. 9, it is possible to prevent the rod-shaped body 100 from impairing a field of view. That is, the surgical instrument 1 according to the embodiment 1 can have the structure that allows the surgical instrument 1 to easily ensure a surgical field also in a narrow and deep part.

(2-5) The distal end of the treatment unit 10 is located at the position away in the radial direction from the longitudinal direction line LL. For example, the distal end sides of the treatment members 11, 12 are bent so as to be away from the longitudinal direction line LL as the distal end sides of the treatment members 11, 12 extend toward the distal ends of the treatment members 11, 12. Accordingly, for example, in a case where an operation in neurosurgery illustrated in FIG. 9 or the like is performed, even when a user (doctor) watches from a direction that makes a shallow angle with respect to the longitudinal direction line LL of the surgical instrument 1 (rod-shaped body 100), a narrow and deep portion that is located on the distal end side also easily enters in his/her field of view. Accordingly, the user can perform the surgical procedure more accurately.

### Embodiment 2

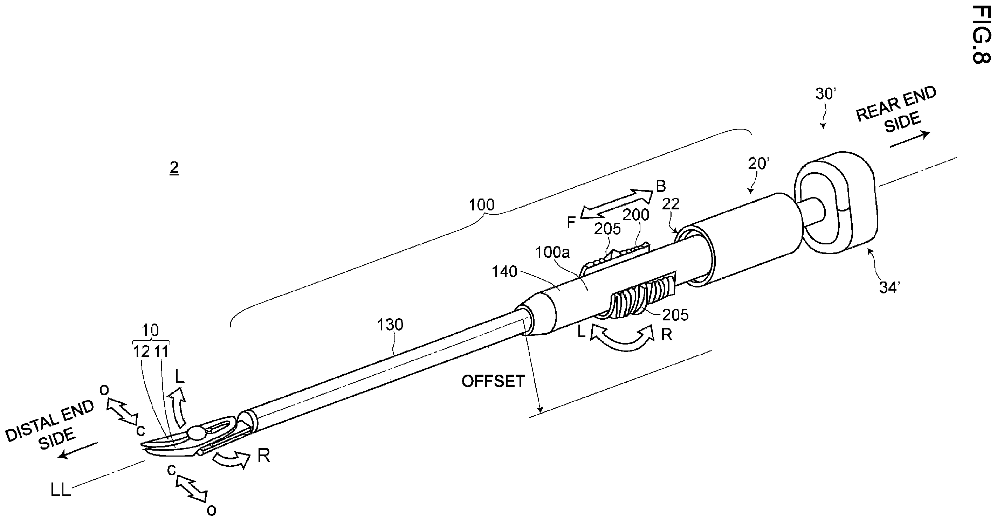

FIG. 8 is a perspective view of a surgical instrument 2 according to the embodiment 2. With respect to constitutional elements having the same basic configuration and technical features as the embodiment 1, symbols used in the embodiment 1 are used also in the embodiment 2, and the description of these constitutional elements is omitted.

Although the surgical instrument 2 according to the embodiment 2 has basically substantially same configuration as the surgical instrument 1 according to the embodiment 1, the surgical instrument 2 according to the embodiment 2 differs from the surgical instrument 1 according to the embodiment 1 with respect to the configuration of a gripping portion. That is, a gripping portion 30' of the surgical instrument 2 is formed such that a palm contact portion 34' is disposed on a rear end side of a fixed cylindrical portion 20', and does not have the finger engaging portion 32 that the gripping portion 30 of the embodiment 1 has (see FIG. 8). The palm contact portion 34' may be directly and continuously formed with the fixed cylindrical portion 20', or may be connected to the fixed cylindrical portion 20' by way of a stay in the same manner as the palm contact portion 34 of the embodiment 1.

It is needless to say that, also in the surgical instrument 2, in the same manner as the surgical instrument 1 according to the embodiment 1, a treatment unit 10 is rotated by rotating a slider handle 200, and a distal end (a pair of treatment members 11, 12) of the treatment unit 10 is opened/closed by advancing or retracting the slider handle 200.

The surgical instrument 2 according to the embodiment 2 has basically the substantially same configuration as the surgical instrument 1 according to the embodiment 1 with respect to the points other than the configuration of the gripping portion. Accordingly, the surgical instrument 2 according to the embodiment 2 has advantageous effects corresponding to the advantageous effects amongst all advantageous effects that the surgical instrument 1 according to the embodiment 1 has.

The present invention has been described based on the respective embodiments described heretofore. However, the present invention is not limited to the above-mentioned respective embodiments. The present invention can be carried out in various modes without departing from the gist of the present invention, and, for example, the following modifications are also conceivable.
(1) In the above-mentioned embodiments, the description has been made by taking the surgical instruments where the distal end sides of the treatment members 11, 12 are bent. However, the present invention is not limited to such a configuration. For example, the treatment members 11, 12 may be formed of a so-called straight-shaped treatment member where the entire treatment portions 11a, 12a of the treatment members 11, 12 extend along the longitudinal direction line LL. In this case, the distal ends of the treatment members 11, 12 are positioned on the same axis as the shaft for manipulating the slider handle 200 and hence, for example, the alignment of the treatment members 11, 12 with a part to be treated can be performed easily.
(2) In the above-mentioned respective embodiments, the description has been made with respect to the case where the open/close converting portion that uses the cam grooves and the bosses is used as the open/close converting portion 103. However, the present invention is not limited to such a configuration. For example, as in the case of the conventional surgical instrument 9 (the needle holder described in patent literature 1), the open/close converting portion 103 may be formed by adopting a mode where a push rod is brought into contact with a mating face of manipulating portions of a pair of treatment members, and a restoring force is imparted from the outside of the manipulating portions by leaf springs.
(3) In the above-mentioned respective embodiments, the description has been made by taking the surgical instrument that can perform a so-called complete manual operation where, when the forward or backward movement of the slider handle 200 is stopped, the degree of opening/closing of the treatment unit 10 is maintained in that state. However, the present invention is not limited to such a surgical instrument, and may be a surgical instrument which can perform a half manual manipulation and a half automatic manipulation. For example, a predetermined spring (for example, a coil spring or the like) that engages with the push rod 110 is disposed in the hollow pipe 130, and the push rod 110, the slider handle 200, the flat plate 120 and the like are automatically advanced toward a distal end side using an elastic force of the spring when a user releases his/her fingers from the slider handle 200 so that the distal end of the treatment unit 10 is automatically closed. As an opposite case, the surgical instrument may be formed such that the push rod 110 and the like are automatically retracted toward a rear end side when the user releases his/her fingers in the same manner so that the distal end of the treatment unit 10 is automatically opened. The invention is defined by the claims that follow.

### Reference Signs List

1,2,9: surgical instrument
10: treatment unit
11, 12: treatment members
11a, 12a: treatment portion
11b, 12b: manipulating portion
11c, 12c: shaft hole
11d, 12d: cam groove
18: pin
18a: pin cap
19: open/close shaft
20, 20': fixed cylindrical portion
22: hole (formed in fixed cylindrical portion)
24: guide groove (formed in the fixed cylindrical portion)
30, 30': gripping portion
32: finger engaging portion
32a: finger engaging surface
34, 34': palm contact portion
34a: palm contact surface
35: stay
36: gripping portion intermediate portion
36a: stay hole
37 stopper screw
81: thumb
82: index finger
83: middle finger
86: palm
87: ball of thumb
88: ball of finger
89: wrist
100: rod-shaped body
100a: outer peripheral surface (of rod shaped body)
101: first connecting portion
102: second connecting portion
103: open/close converting portion
105: guide groove (formed on push rod)
105a, 105b: inner wall (of guide groove)
107: fixing portion of rod-shaped body that fixes open/close shaft
110: push rod
120: flat plate
121: first surface
122: second surface
123: upper boss
124: lower boss
130: hollow pipe
135: treatment unit base
140: barrel
142: slit (of barrel)
142a: inner wall (of slit)
144: flange portion (of barrel)
150: hollow member
152: cutout portion (of hollow member)
152a: inner wall (of cutout portion)
154: engaging groove (in hollow member)
154a: inner protrusion
156: threaded hole (formed in hollow member)
192, 194: screw
200: slider handle
200a: surface of slider handle
205: position marker
210: pawl
212: distal end portion (of pawl)
213: side portion (of pawl)
230: engaging protruding ridge portion
230a: inner groove
230b: side surface (of engaging protruding ridge portion)
254: engaging groove
900: microscope
910: objective lens

## Claims

1. A surgical instrument comprising:
a treatment unit (10) formed of a pair of treatment members (11, 12) having a distal end that is openable and closable;
a rod-shaped body (100) having a rod shape, the rod-shaped body (100) having a distal end side that is connected to the treatment unit (10), and the rod-shaped body (100) extending along a longitudinal direction line;
a fixed cylindrical portion (20) having an approximately cylindrical shape, the fixed cylindrical portion having a hole (22) formed on the distal end side of the fixed cylindrical portion, the hole (22) allowing a rear end side of the rod-shaped body (100) to rotatably engage with the fixed cylindrical portion about the longitudinal direction line by way of an inner wall of the hole; and
a gripping portion (30) integrally provided with the fixed cylindrical portion as a portion that a user grips, wherein
the surgical instrument further comprises a slider handle (200) that is mounted on an outer peripheral surface (100a) of the rod-shaped body (100), and
the slider handle (200) is configured to slide frontward and backward relative to the rod-shaped body (100) in the longitudinal direction line, and to rotate about the longitudinal direction line, whereby
the pair of treatment members (11, 12) of the treatment unit (10) is opened or closed by moving the slider handle (200) forward and backward along the longitudinal direction line, and
the treatment unit (10) is rotatable about the longitudinal direction line by rotating the slider handle (200) about the longitudinal direction line, wherein the slider handle (200) is configured to be manipulated by a thumb (81) and an index finger (82) of a user,
wherein the gripping portion (30) is formed in a protruding manner in a radial direction of the rod-shaped body (100) from the longitudinal direction line, and
the gripping portion (30) includes:
a finger engaging portion (32) having a finger engaging surface (32a) that is a surface with which a finger of the user engages and sets a tangent line thereof in a direction such that the direction intersects with the longitudinal direction line, and the finger engaging portion (32) is disposed at a position that is offset in the radial direction from the longitudinal direction line; and
a palm contact portion (34) that is arranged on the rear end side as viewed from the finger engaging portion (32),
wherein the finger engaging portion (32) is a portion with which at least a middle finger (83) of the user engages.

2. The surgical instrument according to claim 1, wherein the rod-shaped body (100) includes a push rod (110) having a distal end with which the treatment unit engages, and
the surgical instrument further comprises:
a first connecting portion (101) that connects the slider handle (200) and the push rod (110) to each other, and converts frontward and backward movement of the slider handle (200) into frontward and backward movement of the push rod (110);
a second connecting portion (102) that connects the slider handle (200) and a fixed portion of the rod-shaped body (100) to which an open/close shaft (19) of the treatment unit (10) is fixed to each other, and converts rotation of the slider handle (200) into rotation of the treatment unit (10); and
an open/close converting portion (103) that converts frontward and backward movement of the push rod (110) into opening and closing of the treatment unit (10).

3. The surgical instrument according to claim 2, wherein a shaft hole (11c, 12c) is formed in each treatment members (11, 12), the each treatment member (11, 12) includes a treatment portion (11a, 12a) disposed on a more distal end side than the shaft hole (11c, 12c) and a manipulating portion (11b, 12b) disposed on a more rear end side than the shaft hole (11c, 12c),
the pair of treatment members (11, 12) is joined to each other by the open/close shaft (19) that is inserted into the shaft holes (11c, 12c) of the respective treatment members (11, 12) in common,
a cam groove (11d, 12d) that makes a predetermined angle with respect to the longitudinal direction line in a state where the treatment unit (10) is closed is formed in the respective manipulating portions (11b, 12b) respectively,
a flat plate (120) is disposed on a distal end of the push rod (110), an upper boss (123) is formed on a first surface (121) of the flat plate (120), and a lower boss is formed on a second surface (122) of the flat plate (120) that is a surface disposed on a side opposite to the first surface (121),
the upper boss (123) engages with the cam groove (11d, 12d) formed on said one treatment member, and the lower boss engages with the cam groove (11d, 12d) formed in said the other treatment member (12), and
as viewed in cross section on a plane perpendicular to the longitudinal direction line, the manipulating portions (11b, 12b) of the treatment unit (10) and the flat plate fall within an outer diameter of a hollow pipe (130) that houses the push rod (110).

4. The surgical instrument according to any one of claims 1 to 3, wherein a position marker (205) is disposed on a surface of the slider handle (200).

5. The surgical instrument according to any one of claims 1 to 4, wherein a distance between the palm contact portion (34) and the slider handle (200) is changeable.

6. The surgical instrument according to any one of claims 1 to 5, wherein a distal end of the treatment unit (10) is located at a position away from the longitudinal direction line in a radial direction.

## Patentansprüche

1. Chirurgisches Instrument, das umfasst:
eine Behandlungseinheit (10), die aus einem Paar von Behandlungselementen (11, 12) gebildet ist, die ein distales Ende aufweisen, dass geöffnet werden kann und schließbar ist;
einen stabförmigen Körper (100), der eine Stabform aufweist, wobei der stabförmige Körper (100) eine distale Endseite aufweist, die mit der Behandlungseinheit (10) verbunden ist, und wobei sich der stabförmige Körper (100) entlang einer einer Längsrichtungsachse erstreckt;
einen festen zylindrischen Abschnitt (20) mit einer nahezu zylindrischen Form, wobei der feste zylindrische Abschnitt ein Loch (22) aufweist, das an der distalen Endseite des festen zylindrischen Abschnitts gebildet ist, wobei das Loch (22) es einer hinteren Endseite des stabförmigen Körpers (100) ermöglicht, sich bei dem festen zylindrischen Abschnitt um die Längsrichtungsachse mittels einer Innenwand des Lochs drehbar einzurasten; und
ein Greifabschnitt (30), der mit dem festen zylindrischen Abschnitt als ein Abschnitt, den ein Anwender greift, einstückig bereitgestellt ist, wobei
das chirurgische Instrument ferner einen Schiebehandgriff (200) umfasst, der auf einer äußeren Umfangsfläche (100a) des stabförmigen Körpers (100) befestigt ist, und
der Schiebehandgriff (200) so konfiguriert ist, dass er sich in der Längsrichtungsachse vorwärts und rückwärts relativ bezogen auf den stangenförmigen Körpers (100) bewegen und um die Längsrichtungsachse drehen kann, wobei
das Paar der Behandlungselemente (11, 12) der Behandlungseinheit (10) geöffnet oder geschlossen wird, indem der Schiebehandgriff (200) entlang der Längsrichtungsachse vorwärts und rückwärts bewegt wird, und
wobei die Behandlungseinheit (10) um die Längsrichtungsachse drehbar ist, indem der Schiebehandgriff (200) um die Längsrichtungsachse gedreht wird,
wobei der Schiebehandgriff (200) so konfiguriert ist, dass er von einem Daumen (81) und einem Zeigefinger (82) eines Anwenders betätigt werden kann,
wobei der Griffabschnitt (30) in einer Art gebildet ist, die in einer radialen Richtung des stabförmigen Körpers (100) aus der Längsrichtungsachse heraus vorsteht und
der Griffbereich (30) Folgendes aufweist:
einen Fingereingriffsabschnitt (32) mit einer Fingereingriffsoberfläche (32a), die eine Oberfläche ist, mit der ein Finger des Anwenders eingreift und eine Tangentenlinie in einer Richtung derselben derart bestimmt, dass sich die Richtung mit der Längsrichtungsachse schneidet, und wobei der Fingereingriffsabschnitt (32) in einer Position angeordnet ist, die in der radialen Richtung von der Längsrichtungsachse versetzt ist; und
ein Handflächenkontaktabschnitt (34), der auf der hinteren Endseite angeordnet ist, wenn die Betrachtung aus Sicht des Fingereingriffsbereichs (32) erfolgt,
wobei der Fingereingriffsbereich (32) ein Abschnitt ist, mit dem mindestens ein Mittelfinger (83) des Anwenders in Eingriff kommen kann.

2. Chirurgisches Instrument nach Anspruch 1, wobei der stabförmige Körper (100) einen Vorschubstab (110) mit einem distalen Ende aufweist, das mit der Behandlungseinheit im Eingriff ist, und
wobei das chirurgische Instrument ferner umfasst:
einen ersten Verbindungsabschnitt (101), der den Schiebehandgriff (200) und den Vorschubstab (110) miteinander verbindet, und der die Vorwärts- und Rückwärtsbewegung des Schiebehandgriffs (200) in eine Vorwärts- und Rückwärtsbewegung des Vorschubstabs (110) umwandelt;
einen zweiten Verbindungsabschnitt (102), der den Schiebehandgriff (200) und einen festen Abschnitt des stabförmigen Körpers (100) verbindet, an dem ein offener/geschlossener Schaft (19) der Behandlungseinheit (10) gegenseitig befestigt ist, und eine Drehung des Schiebehandgriffs (200) in eine Drehung der Behandlungseinheit (10) umwandelt; und einen Öffnungs-/Schließumwandlungsabschnitt (103), der die Vorwärts- und Rückwärtsbewegung des Vorschubstabs (110) in das Öffnen und Schließen der Behandlungseinheit (10) umwandelt.

3. Chirurgisches Instrument nach Anspruch 2, wobei ein Schaftloch (11c, 12c) in jedem Behandlungselement (11, 12) ausgebildet ist, wobei jedes Behandlungselement (11, 12) einen Behandlungsabschnitt (11a, 12a) aufweist, der sich auf einer entfernteren Endseite als das Schaftloch (11c, 12c) befindet, und wobei sich ein Handhabungsabschnitt (11b, 12b) in einem Bereich befindet, der sich weiter auf der hinteren Endseite als das Schaftlochs (11c, 12c) befindet, wobei
das Paar der Behandlungselemente (11, 12) durch den offenen/geschlossenen Schaft (19) miteinander verbunden ist, der in die Schaftlöcher (11c, 12c) der jeweiligen Behandlungselemente (11, 12) gemeinsam eingesetzt wird,
eine Kurvennut (11d, 12d), die einen vorgegebenen Winkel in Bezug zur Längsrichtungsachse in einem Zustand bildet, in dem die Behandlungseinheit (10) geschlossen ist und in den jeweiligen Handhabungsabschnitten (11b, 12b) jeweils gebildet ist,
eine flache Platte (120) an einem distalen Ende des Vorschubstabs (110) angeordnet ist, wobei ein oberer Vorsprung (123) auf einer ersten Oberfläche (121) der flachen Platte (120) gebildet ist, und ein unterer Vorsprung auf einer zweiten Oberfläche (122) der flachen Platte (120) gebildet ist, die eine Oberfläche ist, die auf einer Seite gegenüber der ersten Oberfläche (121) angeordnet ist,
der obere Vorsprung (123) in die Nockenkurve (11d, 12d), die an dem einen Behandlungselement gebildet ist, eingreift und der untere Vorsprung in die Nockenkurve (11d, 12d), die an dem anderen Behandlungselement (12) gebildet ist, eingreift, und
wie in einer Querschnittsbetrachtung auf einer zur Längsrichtungsachse senkrechten Ebene gesehen werden kann, fallen die Handhabungsabschnitte (11b, 12b) der Behandlungseinheit (10) und die flache Platte in den Bereich innerhalb eines Außendurchmessers eines Hohlrohrs (130), das den Vorschubstab (110) beherbergt.

4. Chirurgisches Instrument nach einem der Ansprüche 1 bis 3, bei dem ein Positionsmarker (205) auf einer Oberfläche des Schiebehandgriffs (200) angeordnet ist.

5. Chirurgisches Instrument nach einem der Ansprüche 1 bis 4, wobei ein Abstand zwischen dem Handflächenkontaktabschnitt (34) und dem Schiebehandgriff (200) veränderbar ist.

6. Chirurgisches Instrument nach einem der Ansprüche 1 bis 5, wobei ein distales Ende der Behandlungseinheit (10) an einer Position abseits der Längsrichtungsachse in einer radialen Richtung angeordnet ist.

## Revendications

1. Instrument chirurgical comprenant :
une unité de traitement (10) formée avec une paire d'éléments de traitement (11, 12) ayant une extrémité distale qui peut s'ouvrir et se fermer ;
un corps en forme de tige (100) ayant une forme de tige, le corps en forme de tige (100) ayant un côté d'extrémité distale qui est raccordé à l'unité de traitement (10), et le corps en forme de tige (100) s'étendant le long d'une ligne de direction longitudinale :
une partie cylindrique fixe (20) ayant une forme approximativement cylindrique, la partie cylindrique fixe ayant un trou (22) formé sur le côté d'extrémité distale de la partie cylindrique fixe, le trou (22) permettant à un côté d'extrémité arrière du corps en forme de tige (100) de se mettre en prise, en rotation, avec la partie cylindrique fixe autour de la ligne de direction longitudinale au moyen d'une paroi interne du trou ; et
une partie de préhension (30) prévue, de manière solidaire, avec la partie cylindrique fixe en tant que partie qu'un utilisateur saisit, dans lequel :
l'instrument chirurgical comprend en outre une poignée à glissière (200) qui est montée sur une surface périphérique externe (100a) du corps en forme de tige (100), et
la poignée à glissière (200) est configurée pour coulisser vers l'avant et vers l'arrière par rapport au corps en forme de tige (100) dans la ligne de direction longitudinale, et pour tourner autour de la ligne de direction longitudinale, moyennant quoi :
la paire d'éléments de traitement (11, 12) de l'unité de traitement (10) est ouverte ou fermée en déplaçant la poignée à glissière (200) vers l'avant et vers l'arrière le long de la ligne de direction longitudinale, et
l'unité de traitement (10) peut tourner autour de la ligne de direction longitudinale en faisant tourner la poignée à glissière (200) autour de la ligne de direction longitudinale,
dans lequel la poignée à glissière (200) est configurée pour être manipulée par un pouce (81) et un index (82) d'un utilisateur,
dans lequel la partie de préhension (30) est formée d'une manière saillante dans une direction radiale du corps en forme de tige (100) à partir de la ligne de direction longitudinale, et
la partie de préhension (30) comprend :
une partie de mise en prise de doigt (32) ayant une surface de mise en prise de doigt (32a) qui est une surface avec laquelle un doigt de l'utilisateur se met en prise et détermine sa ligne tangente dans une direction de sorte que la direction coupe la ligne de direction longitudinale, et la partie de mise en prise de doigt (32) est disposée dans une position qui est décalée dans la direction radiale par rapport à la ligne de direction longitudinale ; et
une partie de contact de paume (34) qui est agencée du côté de l'extrémité arrière, comme observé à partir de la partie de mise en prise de doigt (32),
dans lequel la partie de mise en prise de doigt (32) est une partie avec laquelle au moins un majeur (83) de l'utilisateur se met en prise.

2. Instrument chirurgical selon la revendication 1, dans lequel le corps en forme de tige (100) comprend une tige de poussée (110) ayant une extrémité distale avec laquelle l'unité de traitement se met en prise, et
l'instrument chirurgical comprend en outre :
une première partie de raccordement (101) qui raccorde la poignée à glissière (200) et la tige de poussée (110) entre elles et convertit le mouvement vers l'avant et vers l'arrière de la poignée à glissière (200) en un mouvement vers l'avant et vers l'arrière de la tige de poussée (110) ;
une seconde partie de raccordement (102) qui raccorde la poignée à glissière (200) et une partie fixe du corps en forme de tige (100) à laquelle un arbre ouvert/fermé (19) de l'unité de traitement (10) est fixé à chacune d'entre elles, et convertit la rotation de la poignée à glissière (200) en rotation de l'unité de traitement (10) ; et
une partie de conversion d'ouverture/fermeture (103) qui convertit le mouvement vers l'avant et vers l'arrière de la tige de poussée (110) en ouverture et en fermeture de l'unité de traitement (10).

3. Instrument chirurgical selon la revendication 2, dans lequel un trou d'arbre (11c, 12c) est formé dans chacun des éléments de traitement (11, 12), chaque élément de traitement (11, 12) comprend une partie de traitement (11a, 12a) disposée sur un côté d'extrémité plus distale que le trou d'arbre (11c, 12c) et une partie de manipulation (11b, 12b) disposée sur un côté d'extrémité plus arrière que le trou d'arbre (11c, 12c),
la paire d'éléments de traitement (11, 12) sont assemblés l'un à l'autre par l'arbre d'ouverture/fermeture (19) qui est inséré dans les trous d'arbre (11c,12c) des éléments de traitement (11,12) respectifs en commun,
une rainure de came (11d, 12d) qui fait un angle prédéterminé par rapport à la ligne de direction longitudinale dans un état dans lequel l'unité de traitement (10) est fermée, est formée respectivement dans les parties de manipulation (11b, 12b) respectives,
une plaque plate (120) est disposée sur une extrémité distale de la tige de poussée (110), un bossage supérieur (123) est formé sur une première surface (121) de la plaque plate (120) et un bossage inférieur est formé sur une seconde surface (122) de la plaque plate (120) qui est une surface disposée sur un côté opposé à la première surface (121),
le bossage supérieur (123) se met en prise avec la rainure de came (11d, 12d) formée sur ledit élément de traitement, et le bossage inférieur se met en prise avec la rainure de came (11d, 12d) formée dans ledit autre élément de traitement (12), et
comme observé en coupe sur un plan perpendiculaire à la ligne de direction longitudinale, les parties de manipulation (11b, 12b) de l'unité de traitement (10) et de la plaque plate se trouvent dans un diamètre externe d'un tuyau creux (130) qui loge la tige de poussée (110).

4. Instrument chirurgical selon l'une quelconque des revendications 1 à 3, dans lequel un marqueur de position (205) est disposé sur une surface de la poignée à glissière (200).

5. Instrument chirurgical selon l'une quelconque des revendications 1 à 4, dans lequel une distance entre la partie de contact de paume (34) et la poignée à glissière (200) est modifiable.

6. Instrument chirurgical selon l'une quelconque des revendications 1 à 5, dans lequel une extrémité distale de l'unité de traitement (10) est positionnée dans une position à l'opposé de la ligne de direction longitudinale dans une direction radiale.
